Europäisches Patentamt

(19) European Patent Office

Office européen des brevets

(11) Publication number: **0 236 118**
**A2**

(12) # EUROPEAN PATENT APPLICATION

(21) Application number: **87301827.9**

(22) Date of filing: **03.03.87**

(51) Int. Cl.4: **A 23 K 1/16**
**A 61 K 31/505**

(30) Priority: **04.03.86 US 836266**

(43) Date of publication of application:
**09.09.87 Bulletin 87/37**

(84) Designated Contracting States:
**BE CH DE FR GB IT LI LU NL SE**

(71) Applicant: **PFIZER INC.**
**235 East 42nd Street**
**New York, N.Y. 10017 (US)**

(72) Inventor: **Cornwell, Rendle Lomax**
**27 Pleasant Street**
**Darien Connecticut (US)**

(74) Representative: **Wood, David John et al**
**Pfizer Limited Ramsgate Road**
**Sandwich Kent CT13 9NJ (GB)**

(54) **Method for controlling Strongylus infections in equines.**

(57) Use of pyrantel tartrate for the manufacture of a medicated feed for the control of Strongylus infections in equines by the daily administration to the equines via said feed of at least 2 mg/kg of body weight of pyrantel tartrate at least throughout the grazing season.

EP 0 236 118 A2

**Description**

METHOD FOR CONTROLLING STRONGYLUS INFECTIONS IN EQUINES

This invention relates to a method for controlling internal parasites in equines. More specifically it relates to a method for controlling Strongylus infections in equines by the daily administration to said equines of pyrantel tartrate via their feed.

The most serious equine parasitic diseases are strongylosis in equines of all ages and ascariasis in foals and yearlings. The most harmful of the internal equine parasites are large strongyles, especially Strongylus vulgaris. The damaging effects of said equine parasites are, for the most part, caused by immature larval stages of the parasites rather than by the adult or mature stages.

U.S. Patents 3,549,624 and 3,644,624 issued December 22, 1970 and February 22, 1972, respectively, disclose the anthelmintic activity of pyrantel tartrate and related compounds. Pyrantel tartrate, known chemically as 1,4,5,6-tetrahydro-1-methyl-2-[2-(2-thienyl)ethenyl]pyrimidine tartrate, is sold commercially for use in equines under the trademark Banminth by Pfizer Inc.

The efficacy of pyrantel tartrate as an equine anthelmintic has been reported by many investigators. Representative of, but by no means exhaustive of, such references are the following:

Lyons et al. Am. J. Vet. Res. 35, No. 12, 1515-1522 (1974);

U.S. Patent 3,549,624, issued December 22, 1970;

U.S. Patent 3,644,624, issued February 22, 1972;

Cornwell et al., Vet. Rec., May 25, 1968;

Cornwell et al., Vet. Rec., April 27, 1968;

Cornwell et al., Vet. Rec., August 16, 1969.

The administration of morantel tartrate, a compound structurally related to pyrantel tartrate, to equines to treat Parascarus equorum and Strongylosis infections is reported by Cornwell et al. in Res. Vet. Sci., 14, 134 (1973) and in the Vet. Rec., July 28, 1973, respectively. Its administration on a continual daily basis to livestock, including horses, via their drinking water for the control of internal parasites and to suppress worm-egg or larval output and thus minimize development of larval infection in herbage is disclosed by Downing et al., Irish Vet. J. 221, November, 1974, and in British Patent No. 1,530,161, published October 25, 1978.

Daily low-level feed administration of morantel tartrate to calves from turning out to mid-July was reported by Jones et al.,Brit. Vet. J., 134, 166 (1978) to successfully control parasitic gastroenteritis by reducing contamination of the pasture available to the calves. Continuous low level feed administration of pyrantel tartrate to lambs for prophylaxis of internal parasite infections is reported by Cornwell et al., Brit. Vet. J., 125, 235 and 330 (1969).

Slocombe et al., Can. J. Comp. Med. 44, 93 (1980), reported that high doses of pyrantel pamoate effected a clinical cure of intestinal nematodes in ponies experimentally infected with S. vulgaris larvae, but not a radical cure. At necropsy the ponies were found with severe arteritis and thrombosis of the cranial mesenteric artery and its major branches. Pyrantel hydrochloride was found to effect a radical cure of intestinal nematode infections in equines by Drudge et al., Proc. of the 2nd Intn'l. Conf. on Equine Infectious Diseases, Paris 1969, Vol. 2, pp. 310-322, Basel, Switzerland; S. Karger (1970).

U.S. Patents 4,220,152 and 4,220,153, issued September 2, 1980, describe devices and the administration thereof to ruminants for the controlled release of anthelmintics, including pyrantel tartrate and morantel tartrate, to said ruminants over a prolonged period of time. The devices afford convenient long term protection of ruminants against internal parasites and, at the same time, minimize pasture contamination and reinfestation of ruminants grazing thereon by suppressing helminth egg and larval output.

U.S. Patent 4,228,149 discloses a three-layer non-perforated laminar device for sustained release of anthelmintics, e.g. morantel or pyrantel or a salt thereof, to ruminants.

Copending U.S. application, Serial No. 678,722, filed December 6, 1984 in the name of J. R. Cardinal, describes perforated laminar devices for the controlled and prolonged release of an active agent such as pyrantel tartrate or morantel tartrate to ruminants, and the use of said devices to control helminth infestations in ruminants and on pasture land.

Although continuous release of pyrantel tartrate and of morantel tartrate to ruminants via devices placed in their rumeno-reticular sacs has been described, as has the continuous administration of morantel tartrate to livestock (ruminants and non-ruminants) via their feed or drinking water, there has been no report of the continuous administration of pyrantel tartrate to equines.

Current anthelmintic control programs in equines effectively reduce adult populations of helminths at time of administration, but are only effective if the cycles of all helminths present are synchronized. Even then, the removal of adult helminths and retention of a reservoir of larval stages allows only a short respite in pasture contamination before mature helminths are again present. The animals are, therefore, exposed to risk at all or nearly all times. Further, the problem is generally exacerbated by introduction of new animals on premises occupied by treated animals. No program of treatment or control prior to this invention is capable of keeping the animals free of helminths.

All previously known uses of pyrantel tartrate in equines have been directed to short term treatment of infestations of internal parasites from a therapeutic standpoint. None of the reported studies with pyrantel tartrate, or any other salt or pyrantel, were conducted with the intent of long term control (therapeutic and prophylactic) of internal parasites in equines. If indeed any such use occurred, it was unintended and unappreciated; it was an unrecognized accident. There is, in fact, no known prior use of pyrantel tartrate or other equine anthelmintic which can be considered to have consistently achieved control of strongylosis in equines and especially the prevention of S. vulgaris larval migration.

It has now been surprisingly and unexpectedly found that continuous administration of pyrantel tartrate to equines via their feed during at least the grazing season prevents the migration and establishment of Strongylus vulgaris infections. Mesenteric arterial pathology and equine colic due to S. vulgaris is prevented as a result of larval migration control.

Pyrantel tartrate as used therapeutically in equines effectively removes the following mature parasites: large strongyles (Strongylus vulgaris, S. edentatus and S. equinus); small strongyles; pinworms (Oxyuris equi); ascarids (Parascaris equorum). The therapeutic use does not prevent migration of immature larval stages of said parasites. Its effects are the result of its action upon the adult stages of the parasites.

As used in the method of this invention, pyrantel tartrate is top-dressed or mixed with the equines daily feed so as to provide a level of from about 2 mg/kg to about 10 mg/kg of body weight. Higher levels, e.g. 20 mg/kg, or more, of body weight can be used but are generally avoided for reasons of economy.

When used according to the method of this invention, pyrantel tartrate prevents establishment of helminth infections in equines, reduces the occurrence of damaging lesions of their mesenteric arteries, liver and lungs, and reduces dissemination of the parasites in the equine environment which would otherwise occur.

The efficacy of pyrantel tartrate in controlling the above-enumerated internal parasites in equines and, more importantly, in preventing establishment and migration of Strongylus vulgaris infections in equines is accomplished by daily administration of pyrantel tartrate with the animal's daily feed.

It is preferred, for reasons of convenience and accuracy of administration, to administer the pyrantel tartrate with the animal's daily grain mix to be consumed at one feeding. The pyrantel tartrate is top-dressed or mixed with the animal's daily grain mix ration in amounts sufficient to provide from about 2 to about 10 mg of pyrantel tartrate/kg of the animal's body weight. The favored level of pyrantel tartrate is from about 2 to about 4 mg/kg of the animal's body weight/day. The preferred dose is 2.64 mg/kg of the animal's body weight/day.

Alternatively, the pyrantel tartrate can be administered daily in dry mineral mixtures or pre-mixes containing from 0.01 to about 10% of the anthelmintic agent mixed with salt (sodium chloride) and other minerals with which it is desired to treat the animal. This can then be fed on a daily basis by adjusting the proportion thereof in the mixture to the average daily consumption per animal so as to provide the proper daily dose as specified above. If prepared feed supplements are employed, the material can be administered in admixture with the feed. Again, a concentration range of about 0.01 to 10% of the drug in the feed is employed. However, higher proportions can be satisfactorily employed depending upon the palatability of the product to the animal. This can be readily determined by simple experimentation. It is generally advisable to mix the daily dose with only a portion of the animal's average daily allotment to insure complete consumption of the dose. The balance of his daily feed supplement can then be fed after consumption of the medicated portion in the usual fashion. Additionally, any animal feed composition may be prepared to comprise the usual nutritional balance of energy, proteins, minerals, and vitamins together with the pyrantel tartrate. Some of the various components are commonly grains such as dehydrated alfalfa, ground grain, and grain by-products; animal protein sustances, such as meat and fish by-products; vitaminaceous mixtures, e.g. vitamin A and D mixtures, riboflavin supplements and other vitamin B complexes; and bone meal, limestone, and other inorganic compounds to provide minerals.

The relative proportions of pyrantel tartrate in feeds and feed concentrates may vary somewhat, depending upon the feed with which they are employed. It is advantageously combined in such relative proportions with edible carriers to provide concentrates which may readily be blended with standard nutritionally balanced feeds or which may be used themselves as an adjunct to the normal feedings.

A wide variety of carriers may be employed in the preparation of concentrates containing the aforesaid supplements. Suitable carriers include the following: soybean oil meal, corn gluten meal, cottonseed oil meal, sunflower seed meal, linseed oil meal, cornmeal, dehydrated alfalfa, limestone and corncob meal. The carrier facilitates uniform distribution of the anthelmintic in the finished feed with which the concentrate is blended. This is especially important because only a small proportion of material is required. The concentrate may be surface coated, if desired, with various proteinaceous materials, or edible waxes, such as zein, gelatin, microcrystalline wax and the like to provide a protective film which seals in the active ingredient. It will be appreciated that the proportions of the active ingredient in such concentrates are capable of wide variation since the amount of active material in the finished feed may be adjusted by blending the appropriate proportion of concentrate with the feed to obtain the desired degree of supplementation. In the preparation of high potency concentrates, i.e. premixes, suitable for blending by feed manufacturers to produce finished feeds or concentrates of lower potency, the pyrantel tartrate content may range from about 0.01% to 10% per pound of concentrate as previously noted. The high potency concentrates may be blended by the feed manufacturer with proteinaceous carriers, such as soybean oil meal, to produce concentrated supplements which are suitable for direct feeding to animals. In such instances, the animals are permitted to consume the usual diet of fibrous grains and the like. The concentrates described may also be added to animal feeds to produce a nutritionally balanced, finished feed containing from about 8.0 to about 12,000 g of the anthelmintic per ton of feed.

Of course, daily administration of pyrantel tartrate via boluses, capsules, solutions or suspensions, etc.

containing the appropriate amount of agent can also be done to achieve the surprising results described herein. Such forms of administration are, however, much less convenient than is daily administration via the animal's feed.

The herein-described use of pyrantel tartrate is especially valuable and effective when applied to young and adult equines and to equines maintained in areas to be used by said equines.

The most problematical time of year from the standpoint of heavy pasture contamination in the temperature zones is spring through summer, generally considered to be from about April through September. This time period corresponds approximately to the grazing season. In non-temperate zones, the period of lowest pasture contamination and infectivity normally occurs prior to the rainy season. The equines are generally administered the pyrantel tartrate via their feed two to fourteen days prior to their being placed on pasture.

EXAMPLE 1

This example describes a control efficacy study in worm-free foals.

Foals, from mixed breed mares to include pony and quarter horse types, reared to maintain a worn-free status, were used as the test system. Before foaling, mares were dewormed with injectable ivermectin at 200 mcg/kg. This deworming was followed by fenbendazole paste at 10 mg/kg, at two weeks, then pyrantel pamoate suspension at 6.6 mg/kg, two weeks later. The mares were then housed in equine parasite-free open concrete lots prior to foaling. Fecal exams were conducted to monitor worm-free status of mares. At foaling, normal precautionary measures, such as navel iodine dips, tetanus antitoxin injections and enemas were employed. Foals were weaned at 8-10 weeks of age and moved into a confinement building. Repeated fecal exams were conducted on foals to monitor previous parasite exposure risks.

Twenty-four foals, 84 to 113 days of age and between 45 to 144.2 kg (average weight of 95.3 kg) at initiation of the study were blocked by weight and sex and randomly assigned to one of three groups: T-1 (control) and test groups T-2 and T-3. The study was conducted in two replicates over time with fifteen foals (5 per group) in the first replicate and nine foals (3 per group) in the second replicate. Pyrantel tartrate was fed at levels of 0.0, 2.64 mg/kg and 1.98 mg/kg body weight daily. It was weighed out daily for each animal and placed as a top-dress at the prescribed dosage levels onto a portion of the daily grain mix (a 12% crude protein product) during the morning feeding for 32 days post-challenge with infective larvae. Animals were individually hand fed the top-dress and grain daily. Alfalfa hay and water were available ad libitum throughout the study.

Each animal was infected per os over a four-day period with 250 L3 per day of S. vulgaris and 1,137 embryonated P. equorum eggs per day. In the case of test group T-2 and T-3, the animals were subjected to three days of pyrantel tartrate consumption pre-infection. Individual animal body weights were taken at days 3, 7, 14, 21, 28 and 32; and daily body temperatures on all animals were taken on days 3 through 24 of the study. A peak in average body temperatures serves as a clinical measure that migration of S. vulgaris larvae is occurring as a result of the experimental infection (Duncan, Equine Vet. J. 5, 20-25, 1973; Slocombe et al., Can. J. Comp. Med. 44, 93-100, 1980).

In the present study the two medicated groups (T-2 and T-3) maintained normal body temperature demonstrating that pyrantel tartrate effectively prevented migration of S. vulgaris. The control group reached a peak body temperature at day 9 and elevated temperatures persisted until day 21 as a result of S. vulgaris migration.

Upon completion of the study, the animals were necropsied and examined for lesions and worms in the following manner.

After each animal was stunned and exsanguinated, the abdomen was opened along the ventral midline and the flank cut towards the dorsal side, with care not to sever any major artery. The aorta and its main caudal branch were bluntly dissected from the heart to the kidney branches, with care not to disturb the mesenteric branches. Then, starting at the distal ends of the mesenteric branches, arteries were bluntly dissected towards the root, with care to remove as much fat and tissue as possible. All major arteries from the large intestine were traced from the organ to the main trunks. Liver, kidney and splenic arteries were severed at each organ respectively. After freeing the arterial tree from the attachments, the major artery was cut at the proximal and distal ends and removed to a plastic bucket. After additional cleaning of fat and adhering tissue, the arteries were scored on the amount of adhesion, opened from proximal to distal ends and observations made on lesion score, number of intimal tracks (estimated when numbers were greater than 200), number of larvae observed and recovered from the lesion and the wall thickness of the affected artery measured at the cranial mesenteric artery by use of an electronic caliper. The opened arteries were soaked in saline for 17 hours, with larvae collection at that time. All recovered larvae were counted.

The liver and lungs were removed and the surface of the liver was examined for lesions typical of P. equorum migration. All lesions were counted. Where lesions appeared too numerous to count, a Petri dish ruled with a 36 cm rectangle was used and the lesions in the inscribed area counted. After examination of the lungs for lesions, ten percent of the total weight was minced and floated in saline in a Baermann funnel for four hours. Larvae recovered in this manner were counted and speciated.

The scoring system used as regards adhesion and lesion levels was as follows.

Adhesion score is from 1 to 5 with 1 indicating no deviation from the expected norm. The score is a subjective measure of the deviation from normal and the degree of difficulty encountered in freeing the artery from adjacent tissues.

Lesion score is from 1 to 5 with 1 indicating no apparent pathology. The score is a subjective measure of the deviation from normal and the degree of arteritis and thrombosis. The score also included a value for the number of observed larvae, larvae tracks, and the amount of fibrous debris in the artery.

The scoring system is as follows:

1 = Normal
2 = Mild Lesions
3 = Moderate Lesions
4 = Severe Lesions
5 = Extremely Severe Lesions

## Clinical Observations

Two foals died during the study. Foal #36 (T-1) died due to verminous arteritis and foal #23 (T-2) died of bacterial septicemia (Actinobacillus equii). There were no abnormal clinical observations recorded or deaths that were drug related.

## Necropsy Observations

### Strongylus vulgaris

Adhesion and lesion scores, wall thickness, number of intimal tracks and numbers of S. vulgaris larvae recovered by individual animal at study end (Day 32) are presented in Table 2. Mean treatment values for adhesion and lesion scores, wall thickness, numbers of tracks and larvae recovered for the three treatment groups are presented in Table 1.

Average lesion scores, the measure of damage within the artery, were 4.14, 1.43, and 1.0 (scale of 1 to 5 with 1 = normal) for T-1, T-2, and T-3, respectively. Animal #18 in T-2 accounted for the only abnormal lesion score in the medicated foals. The percent reduction in lesion scores between control and medicated groups was 86.3% (100% if #18 excluded) and 100% for T-2 and T-3, respectively.

Average adhesion scores, the measure of attachment of the artery to adjacent tissues were 4.14, 1.14, 1.13 (scale of 1 to 5 with 1 = normal) for T-1, T-2 and T-3, respectively. The percent reduction in adhesion score between control (T-1) and medicated groups was 95.5% and 95.9% for T-2 and T-3 respectively.

Pyrantel tartrate at 2.64 mg/kg daily was effective in the prevention of S. vulgaris migration based on the lack of pathology in and around the primary site for arterial damage due to S. vulgaris larvae as indicated by the reduction in lesion and adhesion scores of treated vs control animals.

The mean arterial wall thickness for the three treatment groups ranged from 5.52 mm to 1.45 mm. The T-2 and T-3 treatment groups reduced arterial wall thickness by 66.3% (74.3% if #18 excluded) and 73.7%, respectively, over the non-medicated group. Pyrantel tartrate fed daily at 2.64 mg/kg body weight was effective in the prevention of S. vulgaris larvae migration as indicated by the absence of arterial wall thickening associated with S. vulgaris larvae migration.

Intimal tracks of the aorta are an indication that migration of S. vulgaris larvae is actively occurring. In this study the number of tracks were reduced by 97.0% and 81.5% for the T-2 and T-3 groups, respectively, when compared to the non-medicated group. Migration of S. vulgaris as demonstrated by intimal tracks, is prevented when foals are fed pyrantel tartrate at 2.64 mg/kg body weight daily.

The average number of S. vulgaris larvae recovered in the artery for the three treatment groups ranged from 42.86 for the control (T-1) to 0.0 for (T-3). All seven animals in the control group that larvae recovery attempts were made were positive for S. vulgaris. Animal #18 in T-2 accounted for all the larvae recovered in that treatment group (five S. vulgaris larvae). The numbers of larvae recovered were reduced by 98.3% (100% if #18 excluded) and 100% for the T-2 and T-3 groups respectively. The dose 2.64 mg/kg was effective in the prevention of S. vulgaris larvae migration, as demonstrated by the reduction in numbers of larvae recovered from the artery.

### Parascaris equorum

Individual animal P. equorum observations are listed in Table 4. Treatment mean P. equorum are presented in Table 3. Liver lesions were reduced by 52.4% and 60.4% for T-2 and T-3, respectively, when compared to non-medicated controls (T-1).

This study model was acceptable for the evaluation of dose confirmation of pyrantel tartrate at 2.64 mg/kg body weight daily in foals for the prevention of migration of S. vulgaris in that adequate gross pathology (lesion and adhesion scores, wall thickness, larvae recovery and intimal tracks) was demonstrated in the infected control animals; the study was conducted as-scheduled with all protocol-described evaluations being completed, and no circumstance occurred which, in the monitor's opinion, affected the integrity or quality of the data collected.

This study confirms that pyrantel tartrate at the dose of 2.64 mg/kg body weight daily is effective in the prevention of the arterial gross pathology associated with migrating S. vulgaris larvae (arteritis, thrombosis and gross thickening of the arterial wall, larvae, intimal tracks, enlarged lymph nodes and connective tissue) in foals. In addition, pyrantel tartrate, when fed daily at 2.64 mg/kg reduced liver lesions associated with P. equorum migration by more than 50%. All foals gained weight during the study with the exception of animal #33 (T-3) which lost 3.6 kg. This loss was not clinically significant. Average gain of the survivors at end of the study was 17 kg.

### TABLE 1

### SUMMARY OF NECROPSY RESULTS FOR S. VULGARIS

| Treatment | S. Vulgaris Artery Results | | | | |
|---|---|---|---|---|---|
| | Lesion* Score | Adhesion* Score | Wall Thickness (mm) | Number Tracks | Number Larvae |
| T-1 | 4.14 | 4.14 | 5.52 | 67.43 | 42.86 |
| T-2 | 1.43 | 1.14 | 1.86 | 2.00 | 0.71 |
| T-3 | 1.00 | 1.13 | 1.45 | 12.5 | 0.00 |
| % Reduction | | | | | |
| T-1 vs T-2 | 86.3 | 95.5 | 66.3 | 97.0 | 98.3 |
| T-1 vs T-3 | 100 | 95.9 | 73.7 | 81.5 | 100 |

Treatment Description

T-1 - Infected non-medicated controls.

T-2 - Infected 2.64 mg/kg pyrantel tartrate daily.

T-3 - Infected 1.98 mg/kg pyrantel tartrate daily.

*Lesion and adhesion scoring system: 1 = normal; 2 = mild lesion; 3 = moderate lesion; 4 = severe lesion; 5 = extremely severe lesion.

TABLE 1 (Cont.)

1 = Normal

2 = Mild

3 = Moderate

4 = Severe

5 = Extremely severe

## TABLE 2
### INDIVIDUAL ANIMAL NECROPSY RESULTS - S. VULGARIS

| | | | | S. Vulgaris Artery Results | | |
|---|---|---|---|---|---|---|
| Treatment Group | Animal Number | Lesion Score | Adhesion Score | Wall Thickness (mm) | Larvae Recovery | Aorta Tracks |
| T-1 | 1 | 5 | 4 | 3.85 | 17 | 100 |
| T-1 | 4 | 3 | 4 | 3.32 | 27 | 53 |
| T-1 | 13 | 5 | 5 | 6.15 | 2 | 100 |
| T-1 | 29 | 5 | 5 | 8.15 | 87 | 100 |
| T-1 | 99 | 5 | 5 | 6.57 | 166 | 100 |

0 236 118

## TABLE 2 (Cont.)

### S. Vulgaris Artery Results

| Treatment Group | Animal Number | Lesion Score | Adhesion Score | Wall Thickness (mm) | Larvae Recovery | Aorta Tracks |
|---|---|---|---|---|---|---|
| T-1 | 20 | 2 | 2 | 3.24 | 0 | 7 |
| T-1 | 35 | 4 | 4 | 7.38 | 1 | 12 |
| T-1 | *36 | | | | | |
| | Average | 4.14 | 4.14 | 5.52 | 42.86 | 67.43 |
| | Standard Deviation | 1.21 | 1.07 | 2.03 | 62.30 | 43.16 |
| T-2 | 3 | 1 | 2 | 1.7 | 0 | 0 |
| T-2 | 6 | 1 | 1 | 1.45 | 0 | 0 |
| T-2 | 15 | 1 | 1 | 1.41 | 0 | 0 |
| T-2 | 18 | 4 | 1 | 4.49 | 5 | 0 |
| T-2 | *23 | | | | | |
| T-2 | 34 | 1 | 1 | 1.39 | 0 | 0 |

TABLE 2 (Cont.)

| Treatment Group | Animal Number | S. Vulgaris Artery Results | | | | |
|---|---|---|---|---|---|---|
| | | Lesion Score | Adhesion Score | Wall Thickness (mm) | Larvae Recovery | Aorta Tracks |
| T-2 | 42 | 1 | 1 | 1.19 | 0 | 0 |
| T-2 | 47 | 1 | 1 | 1.36 | 0 | 14 |
| | Average | 1.43 | 1.14 | 1.86 | 0.71 | 2.00 |
| | Standard Deviation | 1.13 | 0.38 | 1.17 | 1.89 | 5.29 |
| T-3 | 7 | 1 | 1 | 1.4 | 0 | 0 |
| T-3 | 10 | 1 | 1 | 1.14 | 0 | 0 |
| T-3 | 16 | 1 | 1 | 1.39 | 0 | 0 |
| T-3 | 19 | 1 | 1 | 1.38 | 0 | 0 |
| T-3 | 28 | 1 | 1 | 1.68 | 0 | 0 |
| T-3 | 32 | 1 | 1 | 1.47 | 0 | 0 |
| T-3 | 33 | 1 | 2 | 1.54 | 0 | 100 |

0 236 118

TABLE 2 (Cont.)

| Treatment Group | Animal Number | S. Vulgaris Artery Results | | | | |
|---|---|---|---|---|---|---|
| | | Lesion Score | Adhesion Score | Wall Thickness (mm) | Larvae Recovery | Aorta Tracks |
| T-3 | 40 | 1 | 1 | 1.59 | 0 | 0 |
| Average | | 1.00 | 1.13 | 1.45 | 0.00 | 12.50 |
| Standard Deviation | | 0.00 | 0.35 | 0.16 | 0.00 | 35.36 |

Treatment Description

T-1 - Infected non-medicated control.

T-2 - Infected 2.64 mg/kg pyrantel tartrate daily.

T-3 - Infected 1.98 mg/kg pyrantel tartrate daily.

*Animals died while on test.

TABLE 3

Summary of Necropsy Results for P. Equorum

| Treatment | Liver Lesions | Lung Larvae | Lung Weight (gm) |
|---|---|---|---|
| T-1 | 35.6 | 0.0 | 1333.9 |

0 236 118

TABLE 3 (Cont.)

| Treatment | Liver Lesions | Lung Larvae | Lung Weight (gm) |
|---|---|---|---|
| T-2 | 16.9 | 0.0 | 1318.6 |
| T-3 | 14.1 | 0.0 | 1373.7 |

% Reduction

| | |
|---|---|
| T-1 vs T-2 | 52.4 |
| T-1 vs T-3 | 60.4 |

Treatment Description

T-1 - Infected non-medicated control.
T-2 - Infected 2.64 mg/kg pyrantel tartrate daily.
T-3 - Infected 1.98 mg/kg pyrantel tartrate daily.

0 236 118

## TABLE 4

### Individual Animal Necropsy Results - P. Equorum

| Treatment Group | Animal Number | Liver Lesion | Lung Larvae Recovery | Lung Weight |
|---|---|---|---|---|
| T-1 | 1 | 15 | 0 | 1280 |
| T-1 | 4 | 11 | 0 | 1425 |
| T-1 | 13 | 19 | 0 | 1650 |
| T-1 | 29 | 55 | 0 | 1659 |
| T-1 | 99 | 4 | 0 | 1050 |
| T-1 | 20 | 45 | 0 | 752 |
| T-1 | 35 | 100 | 0 | 1521 |
| T-1 | *36 | | | |
| | Average | 35.57 | 0.00 | 1333.86 |
| | Standard Deviation | 33.95 | 0.00 | 334.42 |

TABLE 4 (Cont.)

| Treatment Group | Animal Number | Liver Lesion | Lung Larvae Recovery | Lung Weight |
|---|---|---|---|---|
| T-2 | 3 | 10 | 0 | 1970 |
| T-2 | 6 | 33 | 0 | 1580 |
| T-2 | 15 | 2 | 0 | 1505 |
| T-2 | 18 | 33 | 0 | 1355 |
| T-2 | *23 | | 0 | |
| T-2 | 34 | 5 | 0 | 881 |
| T-2 | 42 | 4 | 0 | 1101 |
| T-2 | 47 | 31 | 0 | 838 |
| | Average | 16.86 | 0.00 | 1318.57 |
| | Standard Deviation | 14.69 | 0.00 | 407.95 |
| T-3 | 7 | 13 | 0 | 950 |

TABLE 4 (Cont.)

| Treatment Group | Animal Number | Liver Lesion | Lung Larvae Recovery | Lung Weight |
|---|---|---|---|---|
| T-3 | 10 | 11 | 0 | 1320 |
| T-3 | 16 | 13 | 0 | 1750 |
| T-3 | 19 | 29 | 0 | 1430 |
| T-3 | 28 | 5 | 0 | 1640 |
| T-3 | 32 | 6 | 0 | 1172 |
| T-3 | 33 | 17 | 0 | |
| T-3 | 40 | 19 | 0 | 1354 |
| | Average | 14.13 | 0.00 | 1373.71 |
| | Standard Deviation | 7.70 | 0.00 | 270.54 |

Treatment Description

T-1 - Infected non-medicated control.

T-2 - Infected 2.64 mg/kg pyrantel tartrate daily.

T-3 - Infected 1.98 mg/kg pyrantel tartrate daily.

*Animals died while on test.

EXAMPLE 2

This example describes the efficacy of pyrantel tartrate in horses for the prevention of migration of S. vulgaris larvae and the control of large Strongyles, small Strongyles, Oxyuris equi and Parascaris equorum.

Forty mixed breed mares with foals were assigned to one of two groups; a non-medicated control group (T-1) and a medicated group (T-2). Three months prior to start of this study, which ran for 535 days (from July 11, 1983 to December 27, 1984) the mares were dewormed with ivermectin and fenbendazole according to label directions. The mares in the control group (T-1) were also dewormed twice with pyrantel pamoate suspension according to label directions (6.6 mg/kg body weight).

All animals were fed native grass (pasture) supplemented with baled hay ad libitum, and grain mix daily. The grain mix was a 12% crude protein product commercially available. The test animals (group T-2) received pyrantel tartrate at a level of 2.64 mg/kg of body weight as a top-dress on their daily grain mix during the morning feeding. The pyrantel tartrate was supplied in alfalfa pellets containing 9600 gm of pyrantel tartrate per ton. The test group animals were placed in individual stalls for their morning feeding and given three hours to consume their grain mix plus pyrantel tartrate. Uneaten food and drug were recorded. Test animals were weighed at 27-30 day intervals throughout the study as a measure of their clinical condition and for adjusting the pyrantel tartrate dose.

Test and control animals were grazed on a twenty acre native grass pasture that had not been grazed by horses for at least 20 years. Prior to initiation of the study a band of ten ponies known to be infected with large and small Strongyles was allowed to graze the pasture for two weeks. The pasture was divided into two equal pastures, one for the control group and one for the test group. Animals were sacrificed at the end of each grazing season for the purpose of determining critical parasite worm counts. In 1983, two mares and three foals from the control (T-1) group died shortly before the scheduled necropsies and were used as part of the critical worm count collections. In addition, six mares and six foals from the medicated (T-2) group and four mares and four foals from the control group were sacrificed in November as scheduled. In 1984, six mares and six foals from the control group (T-1) and six mares and five foals from the treated group (T-2) were sacrificed over a three-day period beginning November 5, 1984. The selection of mares for critical worm counts was random from the pool of open mares. A total of 24 mares and 24 foals was sacrificed over the 18 month test period. The necropsy procedure followed that reported in Example 1.

This example demonstrates the efficacy of pyrantel tartrate at 2.64 mg/kg body weight daily in mares and foals for the prevention of S. vulgaris larvae migration and for the control of infections of Large Strongyles, Small Strongyles, Oxyuris equi and Parascaris equorum. Adequate arterial gross pathology (lesion and adhesion scores, wall thickness, larvae recovery and intimal tracks) was demonstrated in the non-medicated control animals. Adequate worm species, distribution and numbers were present in the control animals to evaluate the efficacy of the drug.

Administration of pyrantel tartrate to mares and foals at the dose of 2.64 mg/kg body weight is highly efficacious in the prevention of S. vulgaris migration based on the reduction of pathology in and around the primary site for arterial damage due to S. vulgaris larvae as indicated by the reduction in lesion and adhesion scores. Furthermore, arterial wall thickening associated with S. vulgaris larvae migration and the intimal tracks, which are an indication that migration is actively occurring, were reduced. Finally, migration was prevented as demonstrated by the reduction in the numbers of S. vulgaris larvae recovered from the artery.

The administration of pyrantel tartrate to mares and foals at the dose of 2.64 mg/kg body weight daily is highly efficacious for the control of adult Large Strongyles, 100.0%; control of adult and fourth-stage larvae of Small Strongyles, 99.8%; control of adult and fourth-stage larvae of Oxyuris equi, 98.2%; and control of adult and fourth-stage larvae of P. equorum, 100.0%. There were no adverse effects of feeding the test article daily for up to 535 days in the health or performance of mares and foals.

EXAMPLE 3

This example, essentially a repeat of Example 2, describes results of a field study conducted with a complete feed dosage form of pyrantel tartrate, a grain mix containing 1000 gm of pyrantel tartrate per ton.

A total of 64 mares with foals, divided into two equal groups, a control (T-1) and a test (T-2) group, were acclimated to the diet prior to initiation of the study. The diet consisted of native grass pasture supplemented with baled hay (ad libitum) and a standard commercially available grain mix. Each group was pastured on pastures previously seeded with manure known to contain strongyle (both large and small) eggs.

This example further demonstrates the efficacy of pyrantel tartrate at 2.64 mg/kg body weight daily in mares and foals for the prevention of S. vulgaris larvae migration and for the control of infections of large strongyles, small strongyles, Oxyuris equi and Parascaris equorum. Adequate arterial gross pathology was demonstrated in the non-medicated control animals. Adequate worm species, distribution and numbers were present in the control animals to evaluate the efficacy of the drug.

The administration of pyrantel tartrate to mares and foals at the dose of 2.64 mg/kg body weight is highly efficacious in the prevention of S. vulgaris migration and in reducing migration of S. vulgaris larvae.

The administration of pyrantel tartrate to mares and foals at the dose of 2.64 mg/kg body weight daily is highly efficacious for the control of adult large strongyles, 99.9%; control of adult and fourth-stage larvae of small strongyles, 98.4%; control of adult and fourth-stage larvae of Oxyuris equi, 92.3%; and control of adult and fourth-stage larvae of P. equorum, 100.0%. There were no adverse effects of feeding the test article daily for up to 534 days in the health or performance of mares and foals.

## Claims

1. Use of pyrantel tartrate for the manufacture of a medicated feed for the control of <u>Strongylus</u> infections in equines which comprises the daily addition to the equines' feed of at least 2 mg/kg of body weight of pyrantel tartrate throughout the grazing season.

2. Use of pyrantel tartrate for the manufacture of a medicated feed for preventing the migration of <u>Strongylus</u> <u>vulgaris</u> larvae in equines which comprises the daily addition to the equines' feed of at least 2 mg/kg of body weight of pyrantel tartrate throughout the grazing season.

3. A use according to claim 1 or 2, which comprises the daily addition of the equines' feed of from 2 to 10 mg/kg of body weight of pyrantel tartrate.

4. Use according to any one of claims 1 to 3 wherein the pyrantel tartrate is mixed with the feed, or wherein the feed is top-dressed with the pyrantel tartrate.

5. Use of pyrantel tartrate for the manufacture of a medicated feed for the control of <u>Strongylus</u> infections in equines by the daily administration to the equines via said feed of at least 2 mg/kg of body weight of pyrantel tartrate throughout the grazing season.